(19) Europäisches Patentamt
European Patent Office
Office européen des brevets

(11) Veröffentlichungsnummer: **0 101 918**
**A1**

(12) # EUROPÄISCHE PATENTANMELDUNG

(21) Anmeldenummer: 83107271.5

(22) Anmeldetag: 25.07.83

(51) Int. Cl.³: C 07 C 69/738
A 61 K 7/48, A 61 K 7/06
C 07 C 103/76

(30) Priorität: 02.08.82 DE 3228842

(43) Veröffentlichungstag der Anmeldung: 07.03.84 Patentblatt 84/10

(84) Benannte Vertragsstaaten: AT BE CH DE FR GB IT LI NL

(71) Anmelder: Henkel Kommanditgesellschaft auf Aktien
Postfach 1100 Henkelstrasse 67
D-4000 Düsseldorf-Holthausen(DE)

(72) Erfinder: Möller, Hinrich, Dr.
Schumannstrasse 11
D-4019 Monheim(DE)

(72) Erfinder: Wallat, Siegfried, Dr.
Marie-Curie-Strasse 9
D-4019 Monheim(DE)

(54) **Sebosuppressive kosmetische Mittel, enthaltend Aryloxobutensäure-Derivate, sowie neue Aryloxobutensäurederivate.**

(57) Die sebosuppressiven Mittel enthalten in geringer Dosierung (0,05-1,0 Gew.%) therapeutisch wirksame 4-Aryl-4-oxo-but-2-ensäure-Derivate der allgemeinen Formel Ar–CO–CH=CH–CO–X.

Ar = ggf. substituierter aromatischer oder heteroaromatischer Rest, vorzugsweise Phenyl, Pyridyl, Thienyl, Furyl oder ein entsprechendes Benzo-Analogon und ggf. substituiert durch Halogen, Alkyl-($C_{1-6}$), Alkoxy-($C_{1-8}$), Hydroxy, Amino, Acylamino-($C_{1-4}$);

X = Alkoxy-($C_{1-10}$) oder eine ggf. durch Aryl oder Alkyl-($C_{1-6}$) substituierte oder den Bestandteil eines Heterocyclus bildende Aminogruppe.

EP 0 101 918 A1

Croydon Printing Company Ltd

Patentanmeldung
D 6637 EP

"Sebosuppressive kosmetische Mittel, enthaltend Aryloxobutensäure-derivate, sowie neue Aryloxobutensäurederivate"

Die Erfindung betrifft topische, kosmetische Mittel zur Verbesserung des fettigen und unästhetischen Aussehens der Haare und der Haut, welche bestimmte Aryloxobutensäure-Derivate enthalten, sowie neue Aryloxobutensäurederivate.

Die moderne Kosmetik ist bemüht, das durch übermäßig starke Absonderung der Talgdrüsen und der Kopfhaut verursachte fettige und wenig ästhetische Aussehen der Haare zu vermindern. Es ist daher vielfach versucht worden, durch geeignete Mittel die Sekretion der Talgdrüsen zu normalisieren, um dem Haar wieder sein gesundes Aussehen zu geben. Es wurden zur Bekämpfung der Seborrhoe des behaarten Kopfes kosmetische Pflegemittel mit Schwefel-, Quecksilber- oder Teerzusatz verwendet. Dabei hat sich gezeigt, daß diese bekannten antiseborrhoischen Zusätze bei längerer Anwendung häufig zu Nebenwirkungen führen, ohne daß wirklich befriedigende Ergebnisse im Hinblick auf Wirksamkeit und anwendungstechnische Eigenschaften erzielt werden konnten. In der DE-OS 29 26 267 werden zur Normalisierung der Fettabsonderung 3,7,11-Trimethyl-2,6,10-dodecatrien-1-ol-Derivate als Zusatz zu kosmetischen Pflegemitteln beschrieben. Es hat sich jedoch gezeigt, daß diese Verbindungen nur eine sehr geringe antiseborrhoische Wirkung besitzen.

...

BAD ORIGINAL

Aufgabe der Erfindung ist es, ein kosmetisches Mittel bereitzustellen, welches gegenüber den bekannten Präparaten eine verstärkte Wirkung, ohne nachteilige Folgen auf den menschlichen Körper, hat.

Es wurde nun überraschend gefunden, daß bestimmte Aryl-oxobutensäure-Derivate hervorragende antiseborrhoische Effekte auch bei sehr geringer Dosierung aufweisen.

Gegenstand der Erfindung sind sebosuppressive kosmetische Mittel, die gekennzeichnet sind durch einen Gehalt an 4-Aryl-4-oxo-but-2-ensäure-Derivaten der allgemeinen Formel

$$Ar - CO - CH = CH - CO - X$$

worin bedeuten

Ar ein gegebenenfalls substituierter aromatischer oder heteroaromatischer Rest,

X eine Alkoxygruppe $C_1$-$C_{10}$ oder eine gegebenenfalls durch Aryl oder Alkyl $C_1$-$C_6$ substituierte oder den Bestandteil eines Heterocyclus bildende Aminogruppe.

Die Reste Ar sind gegebenenfalls substituiertes Phenyl, $\alpha$- oder $\beta$-Naphthyl, Pyridyl, Thienyl, Furyl oder ein entsprechendes Benzo-Analogon. Als Substituenten kommen in Frage Halogen, vorzugsweise Chlor, Alkyl $C_1$-$C_6$, Alkoxy $C_1$-$C_8$, Hydroxy, Amino, Acylamino $C_1$-$C_4$.

Die anspruchsgemäß zu verwendenden Verbindungen können nach allgemein bekannten Verfahren hergestellt werden. Vorzugsweise dienen die Säuren (X = OH) als Ausgangsprodukte, die dann nach üblichen Verfahren in die Ester oder Amide überführt werden können. Die Säuren lassen sich durch Friedel-Crafts-Reaktion der gegebenenfalls

...

BAD ORIGINAL

substituierten Aromaten mit Maleinsäureanhydrid in Gegenwart eines Friedel-Crafts-Katalysators herstellen. Andererseits sind die Ester und Amide auch direkt durch Friedel-Crafts-Reaktion mit ß-Chlorcarbonyl-acrylsäureestern oder -amiden zugänglich. Eine weitere Möglichkeit der Estersynthese stellt die Kondensation von Glyoxylsäureestern mit geeignet substituierten Acetylaromaten oder -heteroaromaten dar.

Im Sinne der Erfindung geeignete Aryloxobutensäurederivate leiten sich beispielsweise ab von folgenden aromatischen oder heteroaromatischen Carbonsäuren:

4-(Phenyl-, 1'-, 2'-Naphthyl-, 4'-Methyl-, 2',4'-, 2',5'-, 3',4'-Dimethyl-, 2',4',6'-, 2',4',5'-Trimethyl-, 4'-Chlor-, 2',4'-, 2',5'-Dichlor-, 2',4',6'-Trichlor-, 4'-Methoxy-, 4'-Butoxy-, 4'-Hexyloxy-, 4'-Octyloxy-, 2',4'-, 2',5'-, 3',4'-Dimethoxy-, 2',3',4'-, 2',4',6'-Trimethoxy-, 4'-t.-Butyl-, 3',4'-Dimethoxy-2'-hydroxy-, 3'-t.-Butyl-2'-hydroxy-, 3',5'-Dit.-butyl-4'-hydroxy-, 4'-Amino-, 2',4'-Diamino-, 4'-Acetylamino-, 4'-Hydroxy-, 4'-Dimethylamino-, 2',6'-Dichlor-4'-methoxy-, 2',3'-Dichlor-4'-methoxy-, 2',4'-Dichlor-5'-methyl, 2',5'-Dichlor-3',6'-dimethyl-, 2'-Chlor-4'-methoxy-, 2'-Chlor-4'-methyl-, 2'-Chlor-4',6'-dimethoxy-, 2'-Chlor-4',5'-dimethyl-phenyl-, 2'-Thienyl-, 3',4'-Dimethyl-3'-thienyl-, 2'-Furyl-, 3'-Pyridyl-)4-oxo-but-2-ensäure.

Als Alkylreste der alkoholischen Komponente der erfindungsgemäß einzusetzenden Esterderivate (X = Alkoxy $C_1$-$C_{10}$) sind zum Beispiel der Methyl-, Ethyl-, Propyl-, Isopropyl-, n-Butyl-, iso-Butyl-, sek. Butyl-, tert.-Butyl-, Pentyl-, Hexyl-, Octyl-, 2-Ethylhexyl-, Decyl-, Benzyl- und Phenacylrest zu nennen.

...

Als Amidkomponente der erfindungsgemäß einzusetzenden Amide (X = substituierte oder ringförmige Aminogruppe) kommen neben primären Aminen zum Beispiel Methyl-, Dimethyl-, Ethyl-, Diethyl-, Propyl-, Dipropyl-, Methylpropyl-, 2-Propyl-, Di-2-propyl-, Butyl-, Dibutyl-, 2-Butyl-, sek. Butyl-, tert.Butyl-, Hexyl-, Dihexyl-, 2-Ethylhexyl-, Octyl-, Ethyloctyl-, Decyl-, Dodecyl-, Ethanol-, Diisopropanol-, 3-Methoxypropyl-, 3-(2-Ethylhexoxy)-propylamid, Benzyl-amid, Anilid, N-Methyl-anilid, Piperidid, 2-Methyl-, 3-Methyl-, 4-Methyl-piperidid, 2,6-Dimethyl-, 3,5-Dimethylpiperidid, Morpholid und 2,6-Dimethylmorpholid in Frage.

Die Verbindungen sind gut haut- und schleimhautverträglich und lassen sich ohne Schwierigkeiten in verschiedene kosmetische Zubereitungen, wie wäßrige oder alkoholische Lösungen, Öle, Suspensionen, Gele, Emulsionen, Salben oder Aerosole einarbeiten. Zur Behandlung von seborrhoischer Haut und fetten Haaren können diese in allen üblichen Applikationsformen, wie Haarwässern, Haarshampoos, Haarkuren, Haarspülungen, Hautlotionen oder Schüttelmixturen eingesetzt werden. Bevorzugt ist die Verwendung in Haarpflegemitteln. Neben der erfindungsgemäßen Wirkstoffkombination können diese kosmetischen Mittel übliche Träger- und Hilfsstoffe, wie Wasser, organische Lösungsmittel, oberflächenaktive Verbindungen, Öle, Fette, Wachse, Duftstoffe, Farbstoffe, Konservierungsmittel und dergleichen enthalten. Die neuen sebosuppressiven Mittel enthalten zweckmäßig 0,01 - 5,0 Gew.-%, vorzugsweise 0,05 - 1 Gew.-% der Aryloxobutensäure-Derivate.

...

Herstellungsbeispiele

A) 4-(4'-Chlor-phenyl)-4-oxo-but-2-ensäure-ethylester

Zu einer Mischung aus 200 ml Chlor-benzol und 18,4 g (138 m Mol) Aluminiumchlorid wurden unter Rühren bei 20 °C 15 g (92 m Mol) Fumarsäure-monoethylesterchlorid innerhalb von 25 Min. getropft. Nach 45-minütigem Erwärmen auf 40 °C wurde abgekühlt, die Mischung auf Eis/Wasser, das 30 ml konzentrierte Salzsäure enthielt, gegossen und in Methylenchlorid aufgenommen und die Lösung eingedampft(am Ende unter vermindertem Druck). Der Rückstand wurde aus Petrolether umkristallisiert. Es wurden 5,7 g reines 4-(4'-Chlorphenyl)-4-oxo-but-2-ensäure-ethylester vom Schmelzpunkt 58 - 60 °C erhalten.

In analoger Weise wurden die folgenden Verbindungen hergestellt, wobei die Reinigung bei flüssigen Stoffen z. T. säulenchromatographisch vorgenommen wurde ($SiO_2/CH_2Cl_2/$ Methylalkohol).

Bei der Herstellung der übrigen substituierten Phenylderivate wurde 1,2-Dichlorethan als Lösungsmittel verwendet.

B) 4-Phenyl-4 oxo-but-2-ensäure-ethylester

Siedepunkt 117 - 119 °C / 0,5 mbar; $n_D^{20}$: 1,5423

C) 4-Phenyl-4-oxo-but-2-ensäure-isopropylester

Siedepunkt 120 - 122 °C / 0,6 mbar; $n_D^{20}$: 1,5321

D) 4-Phenyl-4-oxo-but-2-ensäure-butylester

Siedepunkt 140 °C / 0,9 mbar; $n_D^{20}$: 1,5282

E) 4-(3',4'-Dimethoxy-phenyl)-4-oxo-but-2-ensäure-ethylester

Schmelzpunkt 87 - 89 °C

...

BAD ORIGINAL

F) 4-(4'-Butoxy-phenyl)-4-oxo-but-2-ensäure-ethylester

$n_D^{20}$: 1,5494

G) 4-(4'-Octyloxy-phenyl)-4-oxo-but-2-ensäure-ethylester

Schmelzpunkt 46 - 48 °C

H) 4-(3',4'-Dimethoxy-2'-hydroxy-phenyl)-4-oxo-but-2-ensäure-ethylester

(aus 1,2,3-Trimethoxy-benzol)
Schmelzpunkt 102 - 105 °C

I) 4-(4'-Acetylamino-phenyl)-4-oxo-but-2-ensäure-ethylester

Schmelzpunkt 126 - 129 °C

J) 4-(2',4'-Dichlor-phenyl)-4-oxo-but-2-ensäure-ethylester

Schmelzpunkt 58 - 61 °C

K) 4-Phenyl-4-oxo-but-2-ensäure-diethylamid

$n_D^{20}$: 1,5666

Die antiseborrhoische Wirkung wurde durch nachfolgend beschriebene Tierversuche näher untersucht.

Als Versuchstiere dienten männliche Wistar-Ratten von 220 bis 230 g Körpergewicht. Beurteilt wurde visuell der Bräunungsgrad auf dem Rücken der dort geschorenen Ratten. Die Bräunung wird durch das braune Hautoberflächenlipid der Ratten hervorgerufen. Dieser Test geht von der Beobachtung aus, daß junge weibliche Ratten sowie männliche Ratten nach dem Waschen mit Tensidlösung bzw. einem Lipidlösungsmittel und auch männliche Ratten, die systematisch mit Östrogen behandelt wurden, nur die normale helle, rosafarbene Haut nach dem Scheren aufweisen; parallel dazu sind aus den abgeschnittenen Haaren nur noch vergleichsweise sehr geringe Lipidmengen zu extrahieren.

...

BAD ORIGINAL

Zur Beurteilung der Wirksamkeit wurden die Prüfsubstanzen in alkoholischer Lösung jeweils 6 Ratten halbseitig auf das Rückenfell gepinselt. Die andere Seite wurde nur mit dem Lösungsmittel ohne Wirkstoffe behandelt.

Während der Versuchsdauer von 14 Tagen wurde an insgesamt 9 Tagen einmal appliziert. Zur weiteren Kontrolle diente eine Gruppe von 6 Ratten, die völlig unbehandelt blieben. Am Ende des Versuchs wurden die Tiere am Rücken und an den Flanken geschoren und von einem Beurteilerpanel (6 Personen) unabhängig unter Doppelblindbedingungen visuell abgemustert.

Bewertungsmethoden:

Als 1. Kriterium wurde bewertet, ob die Mehrheit der Beurteiler richtig die behandelte Seite erkannt haben, wobei wie folgt differenziert wurde:

| Zeichen | Anteil der Beurteiler, die eine Wirkung erkannten |
|---|---|
| ++ | 100 % |
| + | > 50 % – 100 % |
| - | ≤ 50 % |

Als 2. Kriterium wurde der Unterschied zwischen rechter und linker Seite gewertet, wobei pro Beurteiler und Tier jeweils 1 Punkt zu vergeben war, und zwar in der Weise, daß die

| | | |
|---|---|---|
| dunklere Seite | mit | 1 Punkt |
| hellere Seite | mit | 0 Punkte und |
| bei Gleichheit beide Seiten | mit | 0,5 Punkte |

benotet wurden.

...

BAD ORIGINAL

Signifikante Differenzen zwischen unbehandelter und behandelter Seite nach der zweiten Bewertungsmethode zeigen die lokale Wirksamkeit einer Substanz an.

Als 3. Kriterium wurden außerdem noch die Intensitätsunterschiede der Brauntöne nach folgender Skala bewertet:

| | |
|---|---|
| 3 Punkte | stark braun |
| 2 Punkte | mittel braun |
| 1 Punkt | schwach braun |
| 0 Punkte | keine Braunfärbung. |

Nach der dritten Bewertungsmethode werden die Punktsummendifferenzen zwischen den unbehandelten Kontrolltieren und jeweils den behandelten und unbehandelten Seiten der Versuchstiere gebildet, wobei wiederum signifikante Differenzen zwischen Kontrolltieren und der behandelten Seite der Versuchstiere die Wirkung einer Substanz deutlich machen.

Parallel dazu ist in der Regel auch ein deutlicher Unterschied zwischen der unbehandelten und der behandelten Seite der Versuchstiergruppen zu sehen. Dieser ist aber nicht immer so deutlich wie der zwischen Kontrolltieren und behandelter Seite, wofür es verschiedene Gründe geben kann, wie zum Beispiel mechanische Substanzübertragung von einer auf die andere Seite oder Lösungsmitteleinfluß.

Zur Differenzierung der Effekte gemäß Beurteilungsmethode 2 und 3 wurde das folgende Schema verwendet:

...

BAD ORIGINAL

| Zeichen | Punktdifferenz |
|---|---|
| ++ | sehr groß ($\geq$ 99,9 % Wahrscheinlichkeit) |
| + | signifikant ($\geq$ 95 % Wahrscheinlichkeit) |
| - | ($<$ 95 % Wahrscheinlichkeit) |

Es wurden die Antioxidantien und Alkohole jeweils allein und sodann unter den gleichen Bedingungen die Kombination von Antioxidans und Alkohol auf sebosuppressive Effektivität untersucht. Die Ergebnisse sind in der Tabelle 1 zusammengefaßt.

Prozentuale Sebumreduktion

Die Sebumreduktion errechnet sich aus der Punktedifferenz in der Weise, daß man den Quotienten aus der Punktedifferenz $\Delta P$ und der Punktezahl für die Kontrolgruppe $P_k$ bildet und den erhaltenen Wert in % angibt.

$$\text{Sebumreduktion} = \frac{\Delta P}{P_k} \cdot 100 \; [\%]$$

...

BAD ORIGINAL

## T a b e l l e

Bewertung der sebosuppressiven Effekte

| Substanz | Konz. (%ig) | Bewertungsmethode 1 | 2 | 3 | Sebumreduktion |
|---|---|---|---|---|---|
| A | 0,25 | ++ | ++ | ++ | 84 |
| B | 0,1 | ++ | ++ | ++ | . |
| C | 0,1 | ++ | ++ | ++ | 68 |
| D | 0,1 | ++ | ++ | ++ | 66 |
| E | 0,1 | ++ | ++ | ++ | 65 |
| F | 0,5 | ++ | ++ | ++ | 42 |
| H | 0,5 | ++ | ++ | ++ | 69 |
| I | 0,1 | ++ | ++ | ++ | 84 |
| J | 0,1 | ++ | ++ | ++ | 27 |

Nachfolgend werden für die erfindungsgemäßen Mittel Rezepturbeispiele gegeben.

### Beispiel 1

Shampoo für fettendes Haar

| | |
|---|---|
| Fettalkohol-$C_{12}$-$C_{14}$-2EO-Sulfat-Na mit 28 % Waschaktivsubstanz | 42,5 Gew.-T. |
| Kokosfettsäureethanolamid | 3,0 Gew.-T. |
| NaCl | 2,0 Gew.-T. |
| $Na_2SO_4$ | 2,0 Gew.-T. |
| Substanz A | 0,1 Gew.-T. |
| DL-α-Tocopherol | 0,1 Gew.-T. |
| Parfümöl | 0,1 Gew.-T. |
| Wasser | 50,2 Gew.-T. |

...

BAD ORIGINAL

## Beispiel 2

### Hautcreme

| | |
|---|---|
| Selbstemulgierendes Gemisch aus Mono/Diglyceriden höherer gesättigter Fettsäuren mit Kaliumstearat (Cutina KD 16(R)) | 16,0 Gew.-T. |
| Cetylstearylalkohol mit ca. 12 Mol Ethylenoxid | 1,0 Gew.-T. |
| 2-Octyldodecanol | 6,0 Gew.-T. |
| Isopropylmyristat | 4,0 Gew.-T. |
| Glycerin | 6,0 Gew.-T. |
| Farnesol | 0,1 Gew.-T. |
| Substanz E | 0,1 Gew.-T. |
| Wasser | 66,8 Gew.-T. |

## Beispiel 3

### Haarkur

| | |
|---|---|
| Glycerinmono- und distearat (Tegin M(R)) | 0,7 Gew.-T. |
| kationisches Tensid | 2,0 Gew.-T. |
| Cholesterin | 0,2 Gew.-T. |
| Sojalecithin | 0,3 Gew.-T. |
| Emulgator (Emulgarde A(R)) | 8,0 Gew.-T. |
| Substanz I | 0,1 Gew.-T. |
| B H T | 0,2 Gew.-T. |
| Parfümöl | 0,3 Gew.-T. |
| Wasser, entsalzt | 88,2 Gew.-T. |

## Patentansprüche

1. Sebosuppressive kosmetische Mittel, gekennzeichnet durch einen Gehalt an 4-Aryl-4-oxo-but-2-ensäure-Derivaten der allgemeinen Formel

$$Ar - CO - CH = CH - CO - X$$

worin bedeuten

Ar ein gegebenenfalls substituierter aromatischer oder heteroaromatischer Rest,

X eine Alkoxygruppe $C_1$-$C_{10}$ oder eine gegebenenfalls durch Aryl oder Alkyl $C_1$-$C_6$ substituierte oder den Bestandteil eines Heterocyclus bildende Aminogruppe.

2. Mittel nach Anspruch 1, worin Ar ein gegebenenfalls substituiertes Phenyl oder Pyridyl, Thienyl, Furyl oder ein entsprechendes Benzo-Analogon ist.

3. Mittel nach Anspruch 1 und 2, worin Ar durch Halogen, Alkyl $C_1$-$C_6$, Alkoxy $C_1$-$C_8$, Hydroxy, Amino, Acylamino $C_1$-$C_4$ substituiert ist.

4. Sebosuppressive kosmetische Mittel nach Anspruch 1 - 3, dadurch gekennzeichnet, daß sie 0,01 bis 5,0 Gew.-%, vorzugsweise 0,05 - 1,0 Gew.-% der 4-Aryl-4-oxo-but-2-ensäure-Derivate enthalten.

5. Verwendung von kosmetischen Mitteln nach Anspruch 1 - 4 zur Behandlung von Seborrhoe.

...

BAD ORIGINAL

6. 4-(3',4'-Dimethoxy-phenyl)-4-oxo-but-2-ensäure-ethylester.

7. 4-(4'-Butoxy-phenyl-)-4-oxo-but-2-ensäure-ethylester.

8. 4-(4'-Octyloxy-phenyl)-4-oxo-but-2-ensäure-ethylester.

9. 4-(3',4'-Dimethoxy-2'-hydroxy-phenyl)-4 oxo-but-2-ensäure-ethylester

10. 4-Phenyl-4-oxo-but-2-ensäure-diethylamid.

BAD ORIGINAL

0101918

Europäisches Patentamt

# EUROPÄISCHER RECHERCHENBERICHT

Nummer der Anmeldung

EP 83 10 7271

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | FR-A-2 481 118 (ROUSSEL-UCLAF) * Seite 1, Zeilen 12-36; Seite 2, Zeilen 1-7; Seite 3, Zeilen 2-15; Seite 6, Beispiel 1; Seite 13, Anspruch 1 * --- | 1 | C 07 C 69/738<br>A 61 K 7/48<br>A 61 K 7/06<br>C 07 C 103/76 |
| A | FR-A-2 480 600 (PIERRE FABRE) * Seite 1, Zeilen 1-9, 12-15; Seite 2, Zeilen 11-18; Seiten 16-17 * ----- | 1 | |
| | | | RECHERCHIERTE SACHGEBIETE (Int. Cl. 3) |
| | | | C 07 C 69/00<br>A 61 K 7/00 |

Der vorliegende Recherchenbericht wurde für alle Patentansprüche erstellt.

| Recherchenort | Abschlußdatum der Recherche | Prüfer |
|---|---|---|
| DEN HAAG | 15-11-1983 | KINZINGER J.M. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82